# EUROPÄISCHE PATENTSCHRIFT

(11) **EP 3 356 490 B1**
(45) Veröffentlichungstag und Bekanntmachung des Hinweises auf die Patenterteilung: **06.03.2019**
(21) Anmeldenummer: 17700433.0
(22) Anmeldetag: 16.01.2017
(51) Int. Cl.: C09J 183/04

(54) **ZU SILIKONGEL VERNETZBARE SILICONZUSAMMENSETZUNG**
SILICONE COMPOSITION CROSSLINKABLE TO GIVE SILICONE GEL
COMPOSITION DE SILICONE POUVANT ÊTRE RÉTICULÉE POUR FORMER UN GEL DE SILICONE

(30) Priorität: 29.01.2016 DE 102016201363
(43) Veröffentlichungstag der Anmeldung: 08.08.2018
(73) Patentinhaber: Wacker Chemie AG, 81737 München (DE)
(72) Erfinder: AUZIAS, Birgit, 84547 Emmerting (DE); HERGERT, Brigitte, 84489 Burghausen (DE); KUHN, Arvid, 84489 Burghausen (DE)
(74) Vertreter: Fritz, Helmut
(86) Internationale Anmeldenummer: PCT/EP2017/050758
(87) Internationale Veröffentlichungsnummer: WO 2017/129429

(56) Entgegenhaltungen:
- EP-A1- 2 395 053
- EP-A1- 2 608 813
- WO-A1-2008/057155

## Beschreibung

Die Erfindung betrifft eine zu Silikongelen vernetzbare Siliconzusammensetzung mit einem Vernetzer mit sehr geringer SiH-Dichte und relativ langer Kettenlänge.

Silikongele sind als Wundadhäsive in der Medizin etabliert. Es besteht ein Bedarf an Produkten mit höherer Haftkraft in Kombination mit rückstandlosem Ablöseverhalten.
Es ist allgemein bekannt, dass sich die Haftkraft eines Silikongels über seine Härte einstellen lässt. Je weicher das Gel, desto höher die Haftkraft. Hierbei wird i.d.R. die Weichheit durch eine geringere Vernetzungsdichte erreicht. Es besteht allerdings die Limitierung darin, dass es bei zu weicher Einstellung zu Kohäsionsversagen und damit zu Rückstandsbildung auf der Haut kommt.

Bekannte Silikonformulierungen für Gele mit adhäsiven Eigenschaften für Anwendungen auf der Haut variieren beispielsweise in Bezug auf Anzahl der SiH-Gruppen pro Molekül im SiH-Vernetzer, Viskosität des SiH-Vernetzers, Anordnung der SiH-Gruppen im SiH-Vernetzer, Verhältnis SiH-Gruppen/VinylGruppen und SiH-Gehalt im SiH-Vernetzer. Derartige Silikonformulierungen sind beispielsweise beschrieben in EP 0737721 A, EP 2608813 A, EP 322118 A, EP 1633830 A und WO 08057155.

In WO 08057155 sind Silikonformulierungen für Gele beschrieben, die neben kurzkettigen SiH-Vernetzern auch langkettige, endständige SiH-Gruppen aufweisende Vernetzer als Kettenverlängerer enthalten. Das SiH/Vinylverhältnis beträgt 0,7 bis 1,5 und das Verhältnis der SiH-Gruppen im Kettenverlängerer zu allen SiH-Gruppen beträgt 0,4 bis 1,0. Der Gehalt an Wasserstoffgruppen in den SiH-Vernetzern liegt im Bereich von 0,03 Gew.-% bis 1,44 Gew.-%.

Gegenstand der Erfindung ist eine zu Silikongelen vernetzbare Siliconzusammensetzung (S) enthaltend
(A) mindestens zwei Alkenylgruppen pro Molekül enthaltendes Polyorganosiloxan mit einer Viskosität bei 25°C von 200 bis 500000 mPa·s,
(B) mindestens zwei SiH-Gruppen pro Molekül enthaltende Organosiliciumverbindung mit einem durchschnittlichen Gehalt von 2,5 bis 3,5 SiH-Gruppen pro Molekül und einem Gehalt von höchstens 0,025 Gew.-% Wasserstoff in den SiH-Gruppen
   und
(C) Hydrosilylierungskatalysator.

Überraschenderweise wurde gefunden, dass in der Siliconzusammensetzung (S) die Organosiliciumverbindung (B) als Vernetzer mit sehr geringer SiH-Dichte und relativ langer Kettenlänge zu deutlich verbesserten Haftungseigenschaften der vernetzten Silicongele führt. Zuvor wurde angenommen, dass die Organosiliciumverbindung (B) in der Formulierung zu sehr geringer Netzwerkdichte und damit unakzeptabel weichen Formulierungen führen würde.

Das Alkenylgruppen enthaltende Polyorganosiloxan (A) ist vorzugsweise aufgebaut aus Einheiten der allgemeinen Formel (1)

R¹ₓR²_{y}SiO_{(4-x-y)/2} (1),

in der
- **R¹**: einen einwertigen, unsubstituierten oder halogen- oder cyanosubstituierten, gegebenenfalls über eine organische zweiwertige Gruppe an Silicium gebundenen C₁-C₁₀-Kohlenwasserstoffrest, der aliphatische Kohlenstoff-Kohlenstoff Mehrfachbindungen enthält,
- **R²**: einen einwertigen, unsubstituierten oder halogen- oder cyanosubstituierten, über SiC-gebundenen C₁-C₁₀-Kohlenwasserstoffrest, der frei ist von aliphatischen Kohlenstoff-Kohlenstoff Mehrfachbindungen
- **x**: die Werte 0, 1, oder 2,
- **y**: die Werte 0, 1, 2 oder 3 bedeuten, mit der Massgabe, dass mindestens zwei Reste **R¹** in jedem Molekül vorhanden sind und der gemittelte Wert von **(x+y)** im Bereich von 1,8 bis 2,5 liegt.

Die Alkenylgruppen **R¹** sind einer Anlagerungsreaktion mit der SiH-funktionellen Organosiliciumverbindung (B) zugänglich. Üblicherweise werden Alkenylgruppen mit 2 bis 6 Kohlenstoffatomen, wie Vinyl, Allyl, Methallyl, 1-Propenyl, 5-Hexenyl, Ethinyl, Butadienyl, Hexadienyl, Cyclopentenyl, Cyclopentadienyl, Cyclohexenyl, vorzugsweise Vinyl und Allyl, verwendet.

Organische zweiwertige Gruppen, über die die Alkenylgruppen **R¹** an Silicium der Polymerkette gebunden sein können, bestehen beispielsweise aus Oxyalkyleneinheiten, wie solche der allgemeinen Formel (2)

-(O)ₘ[(CH₂)ₙO]ₒ- (2),

in der
- **m**: die Werte 0 oder 1, insbesondere 0,
- **n**: Werte von 1 bis 4, insbesondere 1 oder 2 und
- **o**: Werte von 1 bis 20, insbesondere von 1 bis 5 bedeuten.

Die Oxyalkyleneinheiten der allgemeinen Formel (2) sind links an ein Siliciumatom gebunden.

Die Reste **R¹** können in jeder Position der Polymerkette, insbesondere an den endständigen Siliciumatomen, gebunden sein.

Beispiele für unsubstituierte Reste **R²** sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest; Alkenylreste, wie der Vinyl-, Allyl-, n-5-Hexenyl-, 4-Vinylcyclohexyl- und der 3-Norbornenylrest; Cycloalkylreste, wie Cyclopentyl-, Cyclohexyl-, 4-Ethylcyclohexyl-, Cycloheptylreste, Norbornylreste und Methylcyclohexylreste; Arylreste, wie der Phenyl-, Biphenylyl-, Naphthylrest; Alkarylreste, wie o-, m-, p-Tolylreste und Ethylphenylreste; Aralkylreste, wie der Benzylrest, der alpha- und der ß-Phenylethylrest.

Beispiele für substituierte Kohlenwasserstoffreste als Reste **R²** sind halogenierte Kohlenwasserstoffe, wie der Chlormethyl-, 3-Chlorpropyl-, 3-Brompropyl, 3,3,3-Trifluorpropyl und 5,5,5,4,4,3,3-Heptafluorpentylrest sowie der Chlorphenyl-, Dichlorphenyl- und Trifluortolylrest.

**R²** weist vorzugsweise 1 bis 6 Kohlenstoffatome auf. Insbesondere bevorzugt sind Methyl und Phenyl.

Polyorganosiloxan (A) kann auch eine Mischung verschiedener Alkenylgruppen enthaltender Polyorganosiloxane sein, die sich beispielsweise im Alkenylgruppengehalt, der Art der Alkenylgruppe oder strukturell unterscheiden.

Die Struktur der Alkenylgruppen enthaltenden Polyorganosiloxane (A) kann linear, cyclisch oder auch verzweigt sein. Der Gehalt an zu verzweigten Polyorganosiloxanen führenden tri- und/oder tetrafunktionellen Einheiten ist typischerweise sehr gering, vorzugsweise höchstens 20 Mol-%, insbesondere höchstens 0,1 Mol -%.

Besonders bevorzugt ist die Verwendung Vinylgruppen enthaltender Polydimethylsiloxane, der allgemeinen Formel (3)

(ViMe₂SiO_{1/2})₂(ViMeSiO)ₚ(Me₂SiO)_{q} (3),

wobei
- **Vi**: einen Vinylrest und
- **Me**: einen Methylrest bedeuten
und die nichtnegativen ganzen Zahlen **p** und **q** folgende Relationen erfüllen: **p**≥0, 50<**(p+q)**<20000, vorzugsweise 100<**(p+q)**<1500, und 0<(**p**+1)/**(p+q)**<0,2. Insbesondere ist **p** = 0.

Die Viskosität des Polyorganosiloxans (A) beträgt bei 25°C vorzugsweise 300 bis 200000 mPa·s, insbesondere 800 bis 150000 mPa·s.

In einer bevorzugten Ausführungsform besteht ein Teil des Polyorganosiloxans (A) aus Organosiloxanharz (A1), aufgebaut aus Einheiten der allgemeinen Formeln I, II, III und IV

R₃SiO_{1/2} (I),

R₂SiO_{2/2} (II),

RSiO_{3/2} (III),

SiO_{4/2} (IV),

in denen
- **R**: ausgewählt wird aus **R¹**, **R²** und OH,
mit der Massgabe, dass
mindestens 20 Mol-% der Einheiten ausgewählt werden aus Einheiten der allgemeinen Formeln III und IV,
mindestens 2 der Reste **R** Reste **R¹** sind und höchstens 2 Gew.-% der Reste **R** Reste OH sind.

Vorzugsweise bestehen höchstens 20 Gew.-%, insbesondere höchstens 10 Gew.-% des Polyorganosiloxans (A) aus Organosiloxanharz (A1).

Die bevorzugten Reste **R** sind Methyl, Phenyl und Vinyl.

Die Organosiloxanharze (A1) enthalten vorzugsweise mindestens 30 Mol-%, insbesondere mindestens 40 Mol-% und vorzugsweise höchstens 80 Mol-%, insbesondere höchstens 70 Mol-%Einheiten der allgemeinen Formeln III und IV.

Die Organosiloxanharze (A1) sind vorzugsweise MQ-Siliconharze (MQ), welche mindestens 80 Mol-% Einheiten, vorzugsweise mindestens 95 Mol-%, insbesondere mindestens 97 Mol-% Einheiten der allgemeinen Formeln I und IV enthalten. Das durchschnittliche Verhältnis der Einheiten der allgemeinen Formeln I zu IV beträgt vorzugsweise mindestens 0,25, insbesondere mindestens 0,5 und vorzugsweise höchstens 2, insbesondere höchstens 1,5.

Vorzugsweise sind höchstens 1 Gew.-%, insbesondere höchstens 0,5 Gew.-% der Reste **R** Reste OH.

Vorzugsweise sind mindestens 0,01 Gew.-%, besonders bevorzugt mindestens 0,05 Gew.-%, und vorzugsweise höchstens 8 Mol-%, insbesondere höchstens 5 Mol-% der Reste **R** Reste **R¹**.

Das durchschnittliche Molekulargewicht Mn der Organosiloxanharze (A1) beträgt vorzugsweise mindestens 200 g/mol, insbesondere mindestens 1000 g/mol und vorzugsweise höchstens 100000 g/mol, insbesondere höchstens 20000 g/mol.

Die mindestens zwei SiH-Funktionen pro Molekül enthaltende Organosiliciumverbindung (B) ist vorzugsweise aufgebaut aus Einheiten der allgemeinen Formel (4)

HₐR³_{b}SiO_{(4-a-b)/2} (4),

in der
- **R³**: einen einwertigen, unsubstituierten oder halogen- oder cyanosubstituierten, über SiC-gebundenen C₁-C₁₈-Kohlenwasserstoffrest, der frei ist von aliphatischen Kohlenstoff-Kohlenstoff Mehrfachbindungen und
- **a**: die Werte 0, 1, oder 2,
- **b**: die Werte 0, 1, 2 oder 3 bedeuten,
mit der Maßgabe, dass mindestens zwei siliciumgebundene Wasserstoffatome pro Molekül vorhanden sind.

Beispiele für **R³** sind die für **R²** angegebenen Reste. **R³** weist vorzugsweise 1 bis 6 Kohlenstoffatome auf. Insbesondere bevorzugt sind Methyl und Phenyl.

Der Wasserstoffgehalt der Organosiliciumverbindung (B) in den SiH-Gruppen bezieht sich ausschließlich auf die direkt an Siliciumatome gebundenen Wasserstoffatome. Er liegt vorzugsweise im Bereich von 0,002 bis 0,02 Gew.-% Wasserstoff, vorzugsweise von 0,005 bis 0,018 Gew.-% Wasserstoff. Der Wasserstoffgehalt kann mit ¹H-NMR bestimmt werden.

Die Organosiliciumverbindung (B) enthält vorzugsweise mindestens 50 und höchstens 1000 Siliciumatome pro Molekül. Bevorzugt ist die Verwendung von Organosiliciumverbindung (B), die 100 bis 700 Siliciumatome pro Molekül enthält.

Die Organosiliciumverbindung (B) enthält vorzugsweise durchschnittlich von 2,6 bis 3,4 SiH-Gruppen, insbesondere 2,6 bis 3,3 SiH-Gruppen pro Molekül. In einer besonders bevorzugten Ausführungsform enthält die Organosiliciumverbindung (B) 2,6 bis 2,9 SiH-Gruppen pro Molekül.

Die Viskosität der Organosiliciumverbindung (B) beträgt bei 25°C vorzugsweise 500 bis 20000 mPa·s, insbesondere 1000 bis 10000 mPa·s.

Die Struktur der Organosiliciumverbindung (B) kann linear, verzweigt, cyclisch oder netzwerkartig sein.

Besonders bevorzugte Organosiliciumverbindungen (B) sind lineare Polyorganosiloxane der allgemeinen Formel (5)

(HR⁴₂SiO_{1/2})_{c}(R⁴₃SiO_{1/2})_{d}(HR⁴SiO_{2/2})ₑ(R⁴₂SiO_{2/2})_{f} (5),

wobei
**R⁴** die Bedeutungen von **R³** hat und
die nichtnegativen ganzen Zahlen **c, d, e** und **f** folgende Relationen erfüllen: (**c+d**)=2, (**c+e)**≥2 und 50<(**e+f**)<1000.

Vorzugsweise werden auf 100 Gewichtsteile Polyorganosiloxan (A) 2 bis 50 Gewichtsteile insbesondere 5 bis 40 Gewichtsteile Organosiliciumverbindung (B) eingesetzt.

Als Hydrosilylierungskatalysator (C) können alle bekannten Katalysatoren eingesetzt werden, welche die bei der Vernetzung von additionsvernetzenden Siliconzusammensetzungen ablaufenden Hydrosilylierungsreaktionen katalysieren.

Als Hydrosilylierungskatalysatoren (C) werden insbesondere Metalle und deren Verbindungen aus der Gruppe Platin, Rhodium, Palladium, Ruthenium und Iridium eingesetzt.
Vorzugsweise werden Platin und Platinverbindungen verwendet. Besonders bevorzugt werden solche Platinverbindungen, die in Polyorganosiloxanen löslich sind. Als lösliche Platinverbindungen können beispielsweise die Platin-Olefin-Komplexe der Formeln (PtCl₂·Olefin)₂ und H(PtCl₃·Olefin) verwendet werden, wobei bevorzugt Alkene mit 2 bis 8 Kohlenstoffatomen, wie Ethylen, Propylen, Isomere des Butens und Octens, oder Cycloalkene mit 5 bis 7 Kohlenstoffatomen, wie Cyclopenten, Cyclohexen und Cyclohepten, eingesetzt werden. Weitere lösliche Platin-Katalysatoren sind der Platin-Cyclopropan-Komplex der Formel (PtCl₂C₃H₆)₂, die Umsetzungsprodukte von Hexachloroplatinsäure mit Alkoholen, Ethern und Aldehyden bzw. Mischungen derselben oder das Umsetzungsprodukt von Hexachloroplatinsäure mit Methylvinylcyclotetrasiloxan in Gegenwart von Natriumbicarbonat in ethanolischer Lösung. Besonders bevorzugt sind Komplexe des Platins mit Vinylsiloxanen, wie sym-Divinyltetramethyldisiloxan.

Der Hydrosilylierungskatalysator (C) kann in jeder beliebigen Form eingesetzt werden, beispielsweise auch in Form von Hydrosilylierungskatalysator enthaltenden Mikrokapseln, oder Polyorganosiloxanpartikeln.

Der Gehalt an Hydrosilylierungskatalysator (C) wird vorzugsweise so gewählt, dass die Siliconzusammensetzung (S) einen Pt-Gehalt von 0,1 bis 200 Gew.-ppm, insbesondere von 0,5 bis 40 Gew.-ppm besitzt.

Die Siliconzusammensetzung (S) kann mindestens einen Füllstoff (D) enthalten. Nicht verstärkende Füllstoffe (D) mit einer BET-Oberfläche von bis zu 50 m²/g, sind beispielsweise Quarz, Diatomeenerde, Calciumsilikat, Zirkoniumsilikat, Zeolithe, Metalloxidpulver, wie Aluminium-, Titan-, Eisen-, oder Zinkoxide bzw. deren Mischoxide, Bariumsulfat, Calciumcarbonat, Gips, Siliciumnitrid, Siliciumcarbid, Bornitrid, Glas- und Kunststoffpulver. Verstärkende Füllstoffe, also Füllstoffe mit einer BET-Oberfläche von mindestens 50 m²/g, insbesondere 100 bis 400 m²/g sind beispielsweise pyrogen hergestellte Kieselsäure, gefällte Kieselsäure, Aluminiumhydroxid, Ruß, wie Furnace- und Acetylenruß und Silicium-Aluminium-Mischoxide großer BET-Oberfläche.
Die genannten Füllstoffe (D) können hydrophobiert sein, beispielsweise durch die Behandlung mit Organosilanen, Organosilazanen bzw. -siloxanen oder durch Verätherung von Hydroxylgruppen zu Alkoxygruppen. Es kann eine Art von Füllstoff (D), es kann auch ein Gemisch von mindestens zwei Füllstoffen (D) eingesetzt werden.

Vorzugsweise enthalten die Siliconzusammensetzungen (S) mindestens 0 Gew.-%, besonders bevorzugt mindestens 5 Gew.-%, insbesondere mindestens 10 Gew.-% und höchstens 20 Gew.-% Füllstoffanteil (D).

In einer bevorzugten Ausführungsform enthält die Siliconzusammensetzung (S) neben der SiH-funktionellen Organosiliciumverbindung (B) ein lineares Polydiorganosiloxan (E), dessen beide Kettenenden jeweils eine SiH-Gruppe aufweisen und dessen Kette frei von SiH-Gruppen ist. Das Polydiorganosiloxan (E) dient vorzugsweise als Kettenverlängerer.

Vorzugsweise enthält das Polydiorganosiloxan (E) neben den SiH-Gruppen ausschliesslich einwertige, unsubstituierte oder halogen- oder cyanosubstituierte, C₁-C₁₀-Kohlenwasserstoffreste, die frei sind von aliphatischen Kohlenstoff-Kohlenstoff Mehrfachbindungen, besonders bevorzugt C₁-C₆- Kohlenwasserstoffreste, insbesondere Methylreste.

Das Polydiorganosiloxan (E) enthält vorzugsweise mindestens 50 und höchstens 800 Siliciumatome pro Molekül. Bevorzugt ist die Verwendung von Polydiorganosiloxan (E), das 100 bis 700 Siliciumatome pro Molekül enthält.

Die Viskosität der Organosiliciumverbindung (B) beträgt bei 25°C vorzugsweise 200 bis 10000 mPa·s, insbesondere 500 bis 3000 mPa·s.

Pro 100 Gewichtsteile der SiH-funktionellen Organosiliciumverbindung (B) enthält die Siliconzusammensetzung (S) vorzugsweise 0 bis 200 Gewichtsteile, insbesondere 50 bis 150 Gewichtsteile Polydiorganosiloxan (E).

Besonders gute Haftungseigenschaften erreicht man, wenn die SiH-funktionelle Organosiliciumverbindung (B) und gegebenenfalls Polydiorganosiloxan (E) in solchen Mengen in der vernetzbaren Siliconmasse enthalten sind, dass das Molverhältnis aller SiH-Gruppen der Organosiliciumverbindungen (B) und gegebenenfalls Polydiorganosiloxan (E) zu allen Alkenylgruppen in der Siliconzusammensetzung (S) bei 0,3 bis 0,7 insbesondere bei 0,35 bis 0,65 liegt.

Die Siliconzusammensetzung (S) kann wahlweise als weiteren Bestandteil (Z) mögliche Zusätze zu einem Anteil von 0 bis 70 Gew.-%, vorzugsweise 0,0001 bis 40 Gew.-%, enthalten. Diese Zusätze können beispielsweise harzartige Polyorganosiloxane, die von den Polyorganosiloxanen (A) und (B) verschieden sind, Haftvermittler, Pigmente, Farbstoffe, Weichmacher, organische Polymere, Hitzestabilisatoren und Inhibitoren sein. Hierzu zählen Zusätze, wie Farbstoffe, Pigmente und pharmazeutisch aktive Wirkstoffe. Des Weiteren können als Bestandteil thixotropierende Bestandteile, wie hochdisperse Kieselsäure oder andere handelsübliche Thixotropieadditive enthalten sein. Auch können als weiterer Bestandteil (Z) zur besseren Vernetzung vorzugsweise maximal 0,5 Gew, besonders bevorzugt maximal 0,3 Gew.-%, insbesondere <0,1% Gew.-% Peroxid vorhanden sein.

Beispiele für einsetzbare Lösungsmittel (L) sind Ether, insbesondere aliphatische Ether, wie Dimethylether, Diethylether, Methyl-*t*-butylether, Diisopropylether, Dioxan oder Tetrahydrofuran, Ester, insbesondere aliphatische Ester, wie Ethylacetat oder Butylacetat, Ketone, insbesondere aliphatische Ketone, wie Aceton oder Methylethylketon, sterisch gehinderte Alkohole, insbesondere aliphatische Alkohole, wie i-Propanol, t-Butanol, Amide wie DMF, aromatische Kohlenwasserstoffe wie Toluol oder Xylol, aliphatische Kohlenwasserstoffe wie Pentan, Cyclopentan, Hexan, Cyclohexan, Heptan, Chlorkohlenwasserstoffe wie Methylenchlorid oder Chloroform.
Lösungsmittel oder Lösungsmittelgemische mit einem Siedepunkt bzw. Siedebereich von bis zu 120° C bei 0,1 MPa sind bevorzugt.

Bevorzugt handelt es sich bei den Lösungsmitteln (L) um aromatische oder aliphatische Kohlenwasserstoffe.

Die Siliconzusammensetzungen (S) werden vorzugsweise in zwei lagerfähige Komponenten aufgeteilt.
Die Komponente 1 enthält Polyorganosiloxan (A), gegebenenfalls Organosiloxanharz (A1) und Hydrosilylierungskatalysator (C). Die Komponente 2 enthält Organosiliciumverbindung (B) und kann auch zusätzlich Polyorganosiloxan (A) und Polydiorganosiloxan (E) enthalten.
Falls Füllstoffe (D) eingesetzt werden sind diese vorzugsweise in beiden Komponenten enthalten.

Zur Vernetzung werden die Komponenten gemischt und vorzugsweise durch Erwärmen, vorzugsweise bei 20 bis 250°C, insbesondere bei 100 bis 150°C vernetzt.

Die aus den Siliconzusammensetzungen (S) durch Vernetzung erhaltenen Silikongele werden vorzugsweise verwendet in medizinischen Anwendungen, beispielsweise als Wundadhäsive oder Hautadhäsive oder im Bereich Elektronik, beispielsweise als Vergussmassen und in Display-Anwendungen, z.B. als Optically Clear Adhesive.

Alle vorstehenden Symbole der vorstehenden Formeln weisen ihre Bedeutungen jeweils unabhängig voneinander auf. In allen Formeln ist das Siliciumatom vierwertig.

In den folgenden Beispielen sind, falls jeweils nicht anders angegeben, alle Mengen- und Prozentangaben auf das Gewicht bezogen, alle Drücke 101,3 kPa (abs.) und alle Temperaturen 20°C.

### Verfahrensweise zur Herstellung der Gele:

Die Gele werden durch Vermischen der einzelnen Komponenten in einer geeigneten Vorkehrung bei Raumtemperatur hergestellt. Die Vernetzung findet in der Regel bei einer Temperatur von 120°C über einen Zeitraum von 15 Minuten bei Lagerung in einem Umlufttrockenschrank statt, kann aber auch bei Raumtemperatur durchgeführt werden.

### Testmethoden:

### Molekülzusammensetzungen:

Die Molekülzusammensetzungen werden mittels Kernresonanzspektroskopie bestimmt (zu Begrifflichkeiten siehe ASTM E 386: Hochauflösende magnetische Kernresonanzspektroskopie (NMR): Begriffe und Symbole), wobei der ¹H-Kern vermessen wird.

### Beschreibung ¹H-NMR Messung

| | |
|---|---|
| Solvent: | CDCl₃, 99,8%d |
| Probenkonzentration: | 50 mg / 1 ml CDCl₃ in 5 mm NMR-Röhrchen |

Messung ohne Zugabe von TMS, Spektrenreferenzierung von Rest-CHCl₃ in CDCl₃ auf 7,24 ppm

| | |
|---|---|
| Spektrometer: | Bruker Avance I 500 oder Bruker Avance HD 500 |
| Probenkopf: | 5 mm BBO-Probenkopf oder SMART-Probenkopf (Bruker) |

### Messparameter:

Pulprog = zg30
TD = 64k
NS = 64 bzw. 128 (abhängig von der Empfindlichkeit des Probenkopfes)
SW = 20,6 ppm
AQ = 3,17 s
D1 = 5 s
SFO1 = 500,13 MHz
O1 = 6,175 ppm

### Processing-Parameter:

SI = 32k
WDW = EM
LB = 0,3 Hz

Je nach verwendetem Spektrometertyp sind eventuell individuelle Anpassungen der Messparameter erforderlich.

### Bestimmung der Viskosität:

Die Viskositäten werden, wenn nicht anders angegeben, an einem Rheometer MCR302 der Fa. Anton Paar, D-Ostfildern gemäß DIN EN ISO 3219 in Rotation mit einem Kegel-Platte Messsystem bestimmt. Die Messungen erfolgen im newtonschen Bereich der Proben. Bei nicht-newtonschem Verhalten der Probe wird die Scherrate mit angegeben. Falls nicht anders angegeben, gelten alle Viskositätsangaben bei 25°C und Normaldruck von 1013 mbar.

### Penetrationsmessung:

Die Penetration des Materials wird in Anlehnung an DIN EN ISO 2137 an einer für 60 Minuten bei 120°C vernetzten Silicongelprobe von 125 g gemessen. Verwendet wird ein 62,5 g schwerer Hohlkegel, der Messzeitraum beträgt 60 Sekunden.

### Verfahrensweise zur Herstellung der Gele:

Für eine anwendungsfertige Formulierung werden die hier beschriebenen Bestandteile aufgeteilt in
Komponente A: Vinylpolymer (A), Platin (C), gegebenenfalls Additive, gegebenenfalls MQ-Harz (A1);
Komponente B: Vernetzer (B), gegebenenfalls Vinylpolymer (A), gegebenenfalls H-Polymer (E), gegebenenfalls MQ-Harz (A1).

Die Gele werden durch Vermischen der Komponenten A und B in einer geeigneten Vorkehrung bei Raumtemperatur hergestellt. Die Vernetzung findet, wenn nicht anders beschrieben, bei einer Temperatur von 120°C über einen Zeitraum von 15 Minuten in einem Umlufttrockenschrank statt.

### Bestimmung der Haftkraft:

### Probenvorbereitung:

Komponente A und B der Formulierungen werden bei Raumtemperatur miteinander vermischt.

Die noch unvernetzte Siliconmasse wird mit Hilfe eines handelsüblichen Kastenrakels auf Polyurethanfolie (Epurex ®) aufgetragen. Die Streifen haben eine Länge von mindestens 140 mm und eine Breite von 25 mm. Die Schichtdicke der Siliconmasse beträgt 0,1 mm.

Anschließend werden die Proben für 15 Minuten bei 120°C vulkanisiert und vor der Vermessung für 2h bei Raumtemperatur gelagert.

### 90-Grad Trennkraftmessung:

Für die in den Tabellen 1 und 2 angegebenen Werte wird die Messung an einem Trennkraftmessgerät der Firma Q-Tec (Gerät: ZPFI.01) durchgeführt. Pro Messung werden jeweils vier Probestreifen auf eine Edelstahlplatte mit blankgeglühter Oberflache (bright annealed surface) aufgeklebt. Angedrückt wird mit einem Handroller von 2 kg Gewicht (vier Züge, zwei in jede Richtung, Abrollgeschwindigkeit: ca. 200 mm/s).

Nach einer 30-minutigen Wartezeit und Lagerung bei Raumtemperatur wird die Trennkraftmessung an allen vier Streifen parallel durchgeführt. Dafür werden die Streifen in einem Winkel von 90-Grad bei einer konstanten Geschwindigkeit von 5 mm pro Sekunde (+/- 0,2 mm pro Sekunde) von der Stahlplatte gezogen und die dazu benötigte Trennkraft gemessen.

### Beispiele:

### Bestandteile:

ViPo (I): α,ω-Dimethylvinylsiloxy-terminiertes Polydimethylsiloxan mit einer Viskosität von 1000 mPa.s und einem Vinylgehalt von 0,12 mmol/g (0,33 Gew.-%).
ViPo (II): α,ω-Dimethylvinylsiloxy-terminiertes Polydimethylsiloxan mit einer Viskosität von 100.000 mPa.s und einem Vinylgehalt von 0,027 mmol/g (0,07 Gew.-%).
HPo: α,ω-Dimethylhydrogensiloxy-terminiertes Polydimethylsiloxan mit einer Viskosität von 1000 mPa.s und einem SiH Gehalt von 0,010 Gew.-%.
Vernetzer 1: Dimethylhydrogensiloxy-terminiertes Poly-(dimethylsiloxy)-(methyl-hydrogensiloxy)- Copolymer mit einer Viskosität von 1360 mPa.s und einem mittleren Wasserstoffgehalt von 0,011 Gew.-% (entspricht im Mittel 2,4 SiH-Funktionen pro Molekül).
Vernetzer 2: Dimethylhydrogensiloxy-terminiertes Poly-(dimethylsiloxy)-(methyl-hydrogensiloxy)- Copolymer mit einer Viskosität von 9450 mPa.s und einem mittleren Wasserstoffgehalt von 0,006 Gew.-% (entspricht im Mittel 2,9 SiH-Funktionen pro Molekül).
Vernetzer 3: Dimethylhydrogensiloxy-terminiertes Poly-(dimethylsiloxy)-(methyl-hydrogensiloxy)- Copolymer mit einer Viskosität von 1350 mPa.s und einem mittleren Wasserstoffgehalt von 0,014 Gew.-% (entspricht im Mittel 2,9 SiH-Funktionen pro Molekül).
Vernetzer 4: Dimethylhydrogensiloxy-terminiertes Poly-(dimethylsiloxy)-(methyl-hydrogensiloxy)- Copolymer mit einer Viskosität von 1330 mPa.s und einem mittleren Wasserstoffgehalt von 0,015 Gew.-% (entspricht im Mittel 3,2 SiH-Funktionen pro Molekül).
Vernetzer 5: Dimethylhydrogensiloxy-terminiertes Poly-(dimethylsiloxy)-(methyl-hydrogensiloxy)- Copolymer mit einer Viskosität von 160 mPa.s und einem mittleren Wasserstoffgehalt von 0,17 Gew.-% (entspricht im Mittel 11,7 SiH-Funktionen pro Molekül.)
Vernetzer 6: Trimethylsiloxy-terminiertes Poly-(dimethylsiloxy)-(methyl-hydrogensiloxy)- Copolymer mit einer
Viskosität von 177 mPa.s und einem mittleren Wasserstoffgehalt von 0,22 Gew.-% (entspricht im Mittel 16 SiH-Funktionen pro Molekül).
Vernetzer 7: Trimethylsiloxy-terminiertes Poly-(dimethylsiloxy)-(methyl-hydrogensiloxy)- Copolymer mit einer Viskosität von 94 mPa.s und einem mittleren Wasserstoffgehalt von 0,46 Gew.-% (entspricht im Mittel 24,0 SiH-Funktionen pro Molekül)
Harz: Me3SiO1/2 / Me2ViSiO1/2 / SiO4/2 (Vi-substituiertes MQ-Harz), mittleres Molekulargewicht Mn = 2100 g/mol, 1,95 Gew. % Vinyl, SiOH Gehalt = 0,5 Gew. %
Katalysator: Platin-1,3-Divinyl-1,1,3,3-tetramethyldisiloxan-Komplex

### Erfindungsgemäße Beispiele:

### Beispiel 1):

82,90 % ViPo (II)
6,25 % HPo
0,70 % Harz
9,50 % Vernetzer 1
Katalysator entspricht 6 ppm bezogen auf Platin

### Beispiel 2):

78,50 % ViPo (II)
6,50 % HPo
0,70 % Harz
13,60 % Vernetzer 2

### Beispiel 3):

89,21 % ViPo(II)
5,30 % HPo
5,30 % Vernetzer 3
Katalysator entspricht 6 ppm bezogen auf Platin

### Beispiel 4):

85,90 % ViPo(II)
3,00 % Harz
11,00 % Vernetzer 3
Katalysator entspricht 6 ppm bezogen auf Platin

### Beispiel 5):

91,00 % ViPo(II)
0,75 % Harz
8,15 % Vernetzer 4
Katalysator entspricht 6 ppm bezogen auf Platin

### Beispiel 6):

55,48 % ViPo (I)
19,28 % ViPo (II)
5,50 % Harz
27,35 % Vernetzer 4
Katalysator entspricht 6 ppm bezogen auf Platin

### Beispiel 7):

85,30 % ViPo (II)
6,50 % HPo
0,70 % Harz
7,50 % Vernetzer 4
Katalysator entspricht 6 ppm bezogen auf Platin

**Tabelle 1: Hafteigenschaften der erhaltenen Gele auf Stahl:**

| Beispiel | 1 | 2 | 3 | 4 | 5 | 6 | 7 |
|---|---|---|---|---|---|---|---|
| Trennkraft in cN/cm | 157 | 184 | 162 | 178 | 186 | 157 | 160 |
| Art des Risses | adhäsiv | adhäsiv | adhäsiv | adhäsiv | adhäsiv | adhäsiv | adhäsiv |
| Penetration (1/10 mm) | 156 | 165 | 187 | 209 | 209 | 168 | 151 |
| SiH pro Vernetzermolekül | 2,4 | 2,9 | 2,9 | 2,9 | 3,2 | 3,2 | 3,2 |
| Gew.-% SiH pro Vernetzermolekül | 0,011 | 0,006 | 0,014 | 0,014 | 0,015 | 0,015 | 0,015 |
| Länge des Vernetzermoleküls | 292 | 645 | 282 | 282 | 286 | 286 | 286 |

### Nicht erfindungsgemäße Beispiele:

### Beispiel N1)

91,40 % ViPo(II)
7,40 % HPo
0,75 % Harz
0,45 % Vernetzer 5
Katalysator entspricht 6 ppm bezogen auf Platin

### Beispiel N2)

91,49 % ViPo(II)
7,42 % HPo
0,75 % Harz
0,345 % Vernetzer 6
Katalysator entspricht 6 ppm bezogen auf Platin

### Beispiel N3)

91,63 % ViPo(II)
7,45 % HPo
0,75 % Harz
0,175 % Vernetzer 7
Katalysator entspricht 6 ppm bezogen auf Platin

**Tabelle 2: Haftungseigenschaften der erhaltenen nicht erfindungsgemäßen Gele auf Stahl und Vergleich zu erfindungsgemäßen Beispielen 1 und 7:**

| Beispiel | 1 | 7 | N1* | N2* | N3* |
|---|---|---|---|---|---|
| Ablösekraft in cN/cm | 157 | 160 | 64 | 66 | 23 |
| Art des Risses | adhäsiv | adhäsiv | adhäsiv | adhäsiv | adhäsiv |
| Penetration in 1/10 mm | 156 | 151 | 146 | 149 | 138 |
| SiH-Einheiten pro Vernetzermolekül | 2,4 | 3,2 | 11,7 | 15,9 | 24,0 |
| Gew% SiH pro Vernetzermolekül | 0,011 | 0,015 | 0,166 | 0,220 | 0,46 |
| SiH-Gehalt der Formulierung gesamt [ppm] | 3340 | 3380 | 3099 | 3133 | 2391 |

| | | | | | |
|---|---|---|---|---|---|
| *nicht erfindungsgemäß | | | | | |

## Patentansprüche

1. Zu Silikongelen vernetzbare Siliconzusammensetzung (S) enthaltend
(A) mindestens zwei Alkenylgruppen pro Molekül enthaltendes Polyorganosiloxan mit einer Viskosität bei 25°C von 200 bis 500000 mPa.s, gemessen gemäß DIN EN ISO 3219 in Rotation mit einem Kegel-Platte Messsystem bei 1013 mbar,
(B) mindestens zwei SiH-Gruppen pro Molekül enthaltende Organosiliciumverbindung mit einem durchschnittlichen Gehalt von 2,5 bis 3,5 SiH-Gruppen pro Molekül und einem Gehalt von höchstens 0,025 Gew.-% Wasserstoff in den SiH-Gruppen
und
(C) Hydrosilylierungskatalysator.

2. Siliconzusammensetzung (S) nach Anspruch 1, bei der das Alkenylgruppen enthaltende Polyorganosiloxan (A) aufgebaut ist aus Einheiten der allgemeinen Formel (1)
R¹ₓR²_{y}SiO_{(4-x-y)/2} (1),
in der
**R¹** einen einwertigen, unsubstituierten oder halogen- oder cyanosubstituierten, gegebenenfalls über eine organische zweiwertige Gruppe an Silicium gebundenen C₁-C₁₀-Kohlenwasserstoffrest, der aliphatische Kohlenstoff-Kohlenstoff Mehrfachbindungen enthält,
**R²** einen einwertigen, unsubstituierten oder halogen- oder cyanosubstituierten, über SiC-gebundenen C₁-C₁₀-Kohlenwasserstoffrest, der frei ist von aliphatischen Kohlenstoff-Kohlenstoff Mehrfachbindungen
**x** die Werte 0, 1, oder 2,
**y** die Werte 0, 1, 2 oder 3 bedeuten, mit der Massgabe, dass mindestens zwei Reste **R¹** in jedem Molekül vorhanden sind und der gemittelte Wert von **(x+y)** im Bereich von 1,8 bis 2,5 liegt.

3. Siliconzusammensetzung (S) nach Anspruch 1 oder 2, bei der das Alkenylgruppen enthaltende Polyorganosiloxan (A) Vinylgruppen enthaltende Polydimethylsiloxane, der allgemeinen Formel (3)
(ViMe₂SiO_{1/2})₂(ViMeSiO)ₚ(Me₂SiO)_{q} (3),
enthält, wobei
**Vi** einen Vinylrest und
**Me** einen Methylrest bedeuten
und die nichtnegativen ganzen Zahlen **p** und **q** folgende Relationen erfüllen: **p**≥0, 50<**(p+q)**<20000, und 0<(**p**+1)/**(p+q)**<0,2.

4. Siliconzusammensetzung (S) nach einem oder mehreren der Ansprüche 1 bis 3, bei der ein Teil des Polyorganosiloxans (A) aus Organosiloxanharz (A1) besteht, das aufgebaut ist aus Einheiten der allgemeinen Formeln I, II, III und IV
R₃SiO_{1/2} (I),
R₂SiO_{2/2} (II),
RSiO_{3/2} (III),
SiO_{4/2} (IV),
in denen
**R** ausgewählt wird aus **R¹**, **R²** und OH,
wobei die Reste **R¹** und **R²** die in Anspruch 2 beschriebenen Bedeutungen aufweisen,
mit der Massgabe, dass
mindestens 20 Mol-% der Einheiten ausgewählt werden aus Einheiten der allgemeinen Formeln III und IV,
mindestens 2 der Reste **R** Reste **R¹** sind und
höchstens 2 Gew.-% der Reste **R** Reste OH sind.

5. Siliconzusammensetzung (S) nach einem oder mehreren der Ansprüche 1 bis 4, bei der die mindestens zwei SiH-Funktionen pro Molekül enthaltende Organosiliciumverbindung (B) aufgebaut ist aus Einheiten der allgemeinen Formel (4)
HₐR³_{b}SiO_{(4-a-b)/2} (4),
in der
**R³** einen einwertigen, unsubstituierten oder halogen- oder cyanosubstituierten, über SiC-gebundenen C₁-C₁₈-Kohlenwasserstoffrest, der frei ist von aliphatischen Kohlenstoff-Kohlenstoff Mehrfachbindungen und
**a** die Werte 0, 1, oder 2,
**b** die Werte 0, 1, 2 oder 3 bedeuten, mit der Maßgabe, dass mindestens zwei siliciumgebundene Wasserstoffatome pro Molekül vorhanden sind.

6. Siliconzusammensetzung (S) nach einem oder mehreren der Ansprüche 1 bis 5, bei der die Organosiliciumverbindung (B) 50 bis 1000 Siliciumatome pro Molekül enthält.

7. Siliconzusammensetzung (S) nach einem oder mehreren der Ansprüche 1 bis 6, bei der die Organosiliciumverbindung (B) lineare Polyorganosiloxane der allgemeinen Formel (5)
(HR⁴₂SiO_{1/2})_{c}(R⁴₃SiO_{1/2})_{d}(HR⁴SiO_{2/2})ₑ(R⁴₂SiO_{2/2})_{f} (5),
enthält, wobei
**R⁴** die Bedeutungen von **R³** gemäß Anspruch 5 hat und
die nichtnegativen ganzen Zahlen **c, d, e** und **f** folgende Relationen erfüllen: (**c+d**)=2, (**c+e)**≥2 und 50<(**e+f**)<1000.

8. Siliconzusammensetzung (S) nach einem oder mehreren der Ansprüche 1 bis 7, welche ein lineares Polydiorganosiloxan (E) enthält, dessen beide Kettenenden jeweils eine SiH-Gruppe aufweisen und dessen Kette frei von SiH-Gruppen ist.

9. Siliconzusammensetzung (S) nach einem oder mehreren der Ansprüche 1 bis 8, bei der als Hydrosilylierungskatalysatoren (C) Metalle und deren Verbindungen aus der Gruppe Platin, Rhodium, Palladium, Ruthenium und Iridium eingesetzt werden.

10. Verwendung der durch Vernetzung der Siliconzusammensetzungen (S) nach einem oder mehreren der Ansprüche 1 bis 9 erhaltenen Silikongele in medizinischen Anwendungen oder im Bereich Elektronik.

## Claims

1. Silicone composition (S) crosslinkable to afford silicone gels containing
(A) polyorganosiloxane containing at least two alkenyl groups per molecule having a viscosity at 25°C of 200 to 500000 mPa·s measured according to DIN EN ISO 3219 in rotation with a cone-plate measuring system at 1013 mbar,
(B) organosilicon compound containing at least two SiH groups per molecule having an average content of 2.5 to 3.5 SiH groups per molecule and a content of not more than 0.025% by weight of hydrogen in the SiH groups
and
(C) hydrosilylation catalyst.

2. Silicone composition (S) according to Claim 1, wherein the alkenyl-containing polyorganosiloxane (A) is constructed from units of general formula (1)
R¹ₓR²_{y}SiO_{(4-x-y)/2} (1),
in which
**R¹** represents a monovalent, unsubstituted or halogen- or cyano-substituted, C₁-C₁₀-hydrocarbon radical optionally bonded to silicon via an organic divalent group and containing aliphatic carbon-carbon multiple bonds,
**R²** represents a monovalent, unsubstituted or halogen- or cyano-substituted, SiC-bonded C₁-C₁₀-hydrocarbon radical free from aliphatic carbon-carbon multiple bonds
**x** represents the values 0, 1 or 2,
**y** represents the values 0, 1, 2 or 3, with the proviso that at least two radicals **R¹** are present in each molecule and the average value of **(x+y)** is in the range from 1.8 to 2.5.

3. Silicone composition (S) according to Claim 1 or 2, wherein the alkenyl-containing polyorganosiloxane (A) contains vinyl-containing polydimethylsiloxanes of general formula (3)
(ViMe₂SiO_{1/2})₂(ViMeSiO)ₚ(Me₂SiO)_{q} (3),
wherein
**Vi** represents a vinyl radical and
**Me** represents a methyl radical
and the non-negative integers **p** and **q** conform to the following relations: **p**≥0, 50<(**p+q**)<20000 and 0<(**p**+1)/(**p+q**)<0.2.

4. Silicone composition (S) according to one or more of Claims 1 to 3, wherein a portion of the polyorganosiloxane (A) is composed of organosiloxane resin (A1) constructed from units of general formulae I, II, III and IV
R₃SiO_{1/2} (I),
R₂SiO_{2/2} (II),
RSiO_{3/2} (III),
SiO_{4/2} (IV),
in which
**R** is selected from **R¹**, **R²** and OH,
wherein the radicals **R¹** and **R²** are as defined in claim 2,
with the proviso that
at least 20 mol% of the units are selected from units of general formulae III and IV,
at least 2 of the radicals **R** are radicals **R¹** and not more than 2% by weight of the radicals **R** are radicals OH.

5. Silicone composition (S) according to one or more of Claims 1 to 4, wherein the organosilicon compound (B) containing at least two SiH functions per molecule is constructed from units of general formula (4)
HₐR³_{b}Sio_{(4-a-b)/2} (4),
in which
**R³** represents a monovalent, unsubstituted or halogen- or cyano-substituted, SiC-bonded C₁-C₁₈-hydrocarbon radical free from aliphatic carbon-carbon multiple bonds and
**a** represents the values 0, 1, or 2,
**b** represents the values 0, 1, 2 or 3, with the proviso that at least two silicon-bonded hydrogen atoms per molecule are present.

6. Silicone composition (S) according to one or more of Claims 1 to 5, wherein the organosilicon compound (B) contains 50 to 1000 silicon atoms per molecule.

7. Silicone composition (S) according to one or more of Claims 1 to 6, wherein the organosilicon compound (B) contains linear polyorganosiloxanes of general formula (5)
(HR⁴₂SiO_{1/2})_{c}(R⁴₃SiO_{1/2})_{d}(HR⁴SiO_{2/2})ₑ(R⁴₂SiO_{2/2})_{f} (5),
wherein
**R⁴** has the same definitions as **R³** according to claim 5 and
the non-negative integers **c, d, e** and **f** fulfill the following relations: (**c+d**)=2, (**c+e)** ≥2 and 50<(**e+f**)<1000.

8. Silicone composition (S) according to one or more of Claims 1 to 7, which contains a linear polydiorganosiloxane (E) whose two chain ends each exhibit an SiH group and whose chain is free from SiH groups.

9. Silicone composition (S) according to one or more of Claims 1 to 8, wherein as hydrosilylation catalysts (C) metals and compounds thereof from the group of platinum, rhodium, palladium, ruthenium and iridium are employed.

10. Use of the silicone gels obtained by crosslinking of the silicone compositions (S) according to one or more of Claims 1 to 9 in medical applications or in the field of electronics.

## Revendications

1. Composition de silicone (S) réticulable en gels de silicone, contenant :
(A) un polyorganosiloxane contenant au moins deux groupes alcényle par molécule, ayant une viscosité à 25 °C de 200 à 500 000 mPa.s, mesurée selon DIN EN ISO 3219 en rotation avec un système de mesure à cône et plaque à 1 013 mbar,
(B) un composé d'organosilicium contenant au moins deux groupes SiH par molécule, ayant une teneur moyenne de 2,5 à 3,5 groupes SiH par molécule et une teneur d'au plus 0,025 % en poids d'hydrogène dans les groupes SiH,
et
(C) un catalyseur d'hydrosilylation.

2. Composition de silicone (S) selon la revendication 1, dans laquelle le polyorganosiloxane (A) contenant des groupes alcényle est formé par des unités de formule générale (1)
R¹ₓR²_{y}SiO_{(4-x-y)/2} (1)
dans laquelle
**R¹** signifie un radical hydrocarboné en C₁-C₁₀ monovalent, non substitué ou substitué par halogène ou cyano, éventuellement relié au silicium par un groupe organique bivalent, qui contient des liaisons multiples carbone-carbone aliphatiques,
**R²** signifie un radical hydrocarboné en C₁-C₁₀ monovalent, non substitué ou substitué par halogène ou cyano, relié par SiC, qui est exempt de liaisons multiples carbone-carbone aliphatiques,
**x** signifie la valeur 0, 1 ou 2,
**y** signifie la valeur 0, 1, 2 ou 3,
à condition qu'au moins deux radicaux **R¹** soient présents dans chaque molécule et que la valeur moyenne de **(x+y)** se situe dans la plage allant de 1,8 à 2,5.

3. Composition de silicone (S) selon la revendication 1 ou 2, dans laquelle le polyorganosiloxane (A) contenant des groupes alcényle contient des polydiméthylsiloxanes contenant des groupes vinyle, de formule générale (3)
(ViMe₂SiO_{1/2})₂(ViMeSiO)ₚ(Me₂SiO)_{q} (3)
dans laquelle
**Vi** signifie un radical vinyle et
**Me** signifie un radical méthyle,
et les nombres entiers non négatifs **p** et **q** satisfont les relations suivantes : **p** ≥ 0, 50 < **(p+q)** < 20 000 et 0 < (**p**+1)/**(p+q)** < 0,2.

4. Composition de silicone (S) selon une ou plusieurs des revendications 1 à 3, dans laquelle une partie du polyorganosiloxane (A) est constituée par une résine d'organosiloxane (Al), qui est formée par des unités de formule générale I, II, III et IV :
R₃SiO_{1/2} (I),
R₂SiO_{2/2} (II),
RSiO_{3/2} (III),
SiO_{4/2} (IV),
dans lesquelles
**R** est choisi parmi **R¹**, **R²** et OH,
les radicaux **R¹** et **R²** ayant les significations décrites dans la revendication 2,
à condition
qu'au moins 20 % en moles des unités soient choisies parmi les unités de formule générale III et IV,
qu'au moins 2 des radicaux **R** soient des radicaux **R¹** et qu'au plus 2 % en poids des radicaux **R** soient des radicaux OH.

5. Composition de silicone (S) selon une ou plusieurs des revendications 1 à 4, dans laquelle le composé d'organosilicium (B) contenant au moins deux fonctions SiH par molécule est formé par des unités de formule générale (4)
HₐR³_{b}SiO_{(4-a-b)/2} (4)
dans laquelle
**R³** signifie un radical hydrocarboné en C₁-C₁₈ monovalent, non substitué ou substitué par halogène ou cyano, relié par SiC, qui est exempt de liaisons multiples carbone-carbone aliphatiques, et
**a** signifie la valeur 0, 1 ou 2,
**b** signifie la valeur 0, 1, 2 ou 3,
à condition qu'au moins deux atomes d'hydrogène reliés au silicium soient présents par molécule.

6. Composition de silicone (S) selon une ou plusieurs des revendications 1 à 5, dans laquelle le composé d'organosilicium (B) contient 50 à 1 000 atomes de silicium par molécule.

7. Composition de silicone (S) selon une ou plusieurs des revendications 1 à 6, dans laquelle le composé d'organosilicium (B) contient des polyorganosiloxanes linéaires de formule générale (5)
(HR⁴₂SiO_{1/2})_{c}(R⁴₃SiO_{1/2})_{d}(HR⁴SiO_{2/2})ₑ(R⁴₂SiO_{2/2})_{f} (5)
dans laquelle
**R⁴** a les significations de **R³** selon la revendication 5, et
les nombres entiers non négatifs **c, d, e** et **f** satisfont les relations suivantes : (**c+d**) = 2, (**c+e**) ≥ 2 et 50 < (**e+f**) < 1 000.

8. Composition de silicone (S) selon une ou plusieurs des revendications 1 à 7, qui contient un polydiorganosiloxane linéaire (E), dont les deux extrémités de chaîne comprennent chacune un groupe SiH et dont la chaîne est exempte de groupes SiH.

9. Composition de silicone (S) selon une ou plusieurs des revendications 1 à 8, dans laquelle des métaux et leurs composés du groupe constitué par le platine, le rhodium, le palladium, le ruthénium et l'iridium sont utilisés en tant que catalyseurs d'hydrosilylation (C).

10. Utilisation des gels de silicone obtenus par réticulation des compositions de silicone (S) selon une ou plusieurs des revendications 1 à 9 dans des applications médicales ou dans le domaine électronique.
